# EUROPEAN PATENT APPLICATION

(11) **EP 4 751 665 A1**
(43) Date of publication of application: **03.06.2026**
(21) Application number: 24845611.3
(22) Date of filing: 23.07.2024
(51) Int. Cl.: A61B 17/58

(54) **WIRE FIXTURE**

(30) Priority: 25.07.2023 JP 2023121164
(71) Applicant: Eureka Tech Innovations Co., Ltd., Osaka-shi, Osaka 545-0037 (JP)
(72) Inventor: MARUKAWA Yudai, Osaka-shi, Osaka 540-0005 (JP)
(74) Representative: Mewburn Ellis LLP
(86) International application number: PCT/JP2024/026273
(87) International publication number: WO 2025/023236

(57) **Abstract**

In a wire fixture attached to a medical wire inserted into a bone, in order to realize a wire fixture in which damage is less likely to occur in a soft tissue even when a soft tissue such as a blood vessel, a nerve, a tendon, or the like exists in the vicinity of an attachment location, the wire fixture 10 includes a ball member 11 formed in a ball shape and having, as a portion through which the medical wire 5 penetrates, a penetration portion 12 extending in a thickness direction of the ball member 11, and wherein the ball member 11 is attached to the medical wire 5 by crushing the ball member 11 and sandwiching the medical wire 5 by an inner surface of the penetrating part 12 in a state where the medical wire 5 penetrates the penetrating part 12.

## Description

### TECHNICAL FIELD

The present invention relates to a wire fixture used in a medical field and the like.

### BACKGROUND ART

As treatment methods for bone fractures and the like, a percutaneous pinning method and an open reduction and internal fixation method are most widely spread. In these treatment methods, after reduction, a Kirschner steel wire called a "K-wire" is inserted into a bone, and internal fixation of the bone is performed. Regarding the Kirschner steel wire, as a state after treatment, there is a case where an insertion part side is left in a linear shape or bent into an L-shape and exposed to a body surface so that it can be easily removed at an outpatient department after bone fusion is obtained, and there is also a case where it is bent into an L-shape and buried subcutaneously so that it can be removed by adding a small incision.

However, as the state after treatment, when the insertion part side of the Kirschner steel wire is left in a linear shape or bent into an L-shape and exposed to the body surface, there is a risk that the Kirschner steel wire is pushed inward of the bone from the insertion part side and advances deeper than an original position immediately after insertion. In such a case, there is a possibility that the Kirschner steel wire becomes difficult to remove, or a sharp tip site of the Kirschner steel wire penetrates the bone to blindly damage a tissue beyond it. Moreover, when the insertion part side of the Kirschner steel wire is bent into an L-shape and exposed to the body surface, there is a possibility that the L-shaped portion gets caught and the Kirschner steel wire comes out. Further, when the insertion part side of the Kirschner steel wire is buried subcutaneously, there is a possibility that the Kirschner steel wire receives a force from an outer surface of a skin and is pushed inward, and the L-shaped portion interferes with and damages a soft tissue such as a surrounding blood vessel, nerve, tendon, or the like.

Since there is a risk that such a problem occurs, it is desirable that the Kirschner steel wire does not move after treatment. Patent Document 1 describes an internal fixator for fracture treatment used for fracture treatment. This internal fixator for fracture treatment includes a Kirschner steel wire penetrated so as to straddle a fracture site with respect to a treatment target site, a pair of staplers holding both ends of the Kirschner steel wire, and a screw set fixing both ends of the Kirschner steel wire on the staplers by mechanical means.

### [Prior Art Technical Document]

### [Patent Document]

[Patent Document 1] Japanese Patent No. 6616767

### DESCRIPTION OF THE DISCLOSURE

### [Problem(s) to be Solved by the Disclosure]

By the way, regarding the conventional wire fixture, there are many corner parts in the stapler and the bolt set used for fixation of a medical wire such as a Kirschner steel wire. Therefore, when a soft tissue such as a blood vessel, a nerve, a tendon, or the like exists in the vicinity of the stapler and the bolt set, there is a risk that the corner parts of the stapler and the bolt set hit the soft tissue and the soft tissue is damaged.

The present invention was made considering such a situation, and therefore its objective is to realize, in a wire fixture attached to a medical wire inserted into a bone, a wire fixture in which damage is less likely to occur in a soft tissue even when a soft tissue such as a blood vessel, a nerve, a tendon, or the like exists in the vicinity of an attachment location.

### [Means for Solving the Problems]

In order to solve the above-described problem, a first invention is a wire fixture for fixing a position of a medical wire inserted into a bone, the wire fixture being configured to be attached to the medical wire, the wire fixture comprising a ball member formed in a ball shape and having, as a portion through which the medical wire penetrates, a penetration portion extending in a thickness direction of the ball member, and
wherein, the ball member is configured to be crushed in a state that the medical wire penetrates the penetration portion so that the medical wire is clamped by an inner surface of the penetration portion and the ball member is attached to the medical wire.

A second invention is the wire fixture according to the first invention, wherein one or more convex portions are formed on the inner surface of the penetration portion.

A third invention is the wire fixture according to the first invention, wherein a pair of concave-convex parts are formed on the inner surface of the penetration portion in regions facing each other in a front view of the penetration portion.

A fourth invention is the wire fixture according to the third invention, wherein the pair of concave-convex parts face each other in a widthwise direction of the penetration portion in the front view.

A fifth invention is the wire fixture according to the first invention, wherein a plurality of protrusions each having a pointed tip are formed on a back surface of the ball member where one end of the penetration portion opens.

A sixth invention is the wire fixture according to the first invention, wherein a thickness dimension of the ball member is smaller than a minimum outer dimension of the ball member in a front view.

A seventh invention is the wire fixture according to the sixth invention, wherein the ball member is formed in an oblate spheroid shape, and the penetration portion is a penetration hole extending in a widthwise direction of the ball member.

### [Effect of the Disclosure]

In the present invention, the ball member configured to be attached to the medical wire by a simple operation of crushing the ball member and sandwiching the medical wire by the inner surface of the penetrating part thereof. And, even if a soft tissue such as a blood vessel, a nerve, a tendon, or the like exists in the vicinity of the attachment location of the ball member, since the ball member is formed in a ball shape, damage is less likely to occur in the soft tissue even if the ball member contacts the soft tissue. According to the present invention, in the wire fixture attached to the medical wire inserted into the bone, it is possible to realize the wire fixture in which damage is less likely to occur in the soft tissue even when the soft tissue exists in the vicinity of the attachment location.

### BRIEF DESCRIPTION OF THE DRAWINGS

Fig. 1(A) is a front view of a ball member of a wire fixture according to the present embodiment viewed from a front side, Fig. 1(B) is a side view of the ball member, and Fig. 1(C) is a front view of a ball member of a form different from Fig. 1(A).
Fig. 2 is a view for explaining a usage method of the wire fixture according to the present embodiment, wherein Fig. 2(A) shows a state where an insertion work of a medical wire into a bone is completed, Fig. 2(B) shows a state where the ball member is moved to a position where the ball member contacts an outer surface of the bone, Fig. 2(C) shows a state of caulking the ball member with pliers, and Fig. 2(D) shows a state of cutting the medical wire.
Fig. 3(A) is a bottom view of a ball member according to Modification 1 of the present embodiment, and Fig. 3(B) is a side view of the ball member.
Fig. 4 is a side view of a ball member according to Modification 2 of the present embodiment.
Fig. 5 is a perspective view of a ball member according to Modification 3 of the present embodiment.
Fig. 6(A) is a front view of the ball member shown in Fig. 5, Fig. 6(B) is a side view of the ball member viewed from a lower side in Fig. 6(A), Fig. 6(C) is a side view of the ball member viewed from a right side in Fig. 6(A), Fig. 6(D) is a cross-sectional view of the ball member at an A-A cutting position in Fig. 6(A), and Fig. 6(E) is a cross-sectional view at a position corresponding to the A-A cutting position regarding a ball member in which a direction where convex parts are lined continuously is different from Fig. 6(A).
Fig. 7 is a view showing a state where a wire fixture according to Modification 4 of the present embodiment is attached to a medical wire.

### MODES FOR CARRYING OUT THE DISCLOSURE

Hereinafter, an embodiment of the present invention will be described in detail by referring to the drawings. Note that the following embodiment is an example of the present invention, and is not intended to be limiting the present invention, its application or the scope of application of the present invention.

The present embodiment is a wire fixture 10 attached to a medical wire 5 in order to fix a position of the medical wire 5 (Kirschner steel wire or the like) inserted into a bone 8. The wire fixture 10 is used at the time of treatment or surgery such as a percutaneous pinning method or an open reduction and internal fixation method.

The wire fixture 10 includes one ball member 11 having a smooth outer surface and a ball shape for one medical wire 5. For a material of the ball member 11, a material (stainless steel, titanium, or the like) which is medically permissible to be buried subcutaneously and which undergoes plastic deformation when an external force is applied is used.

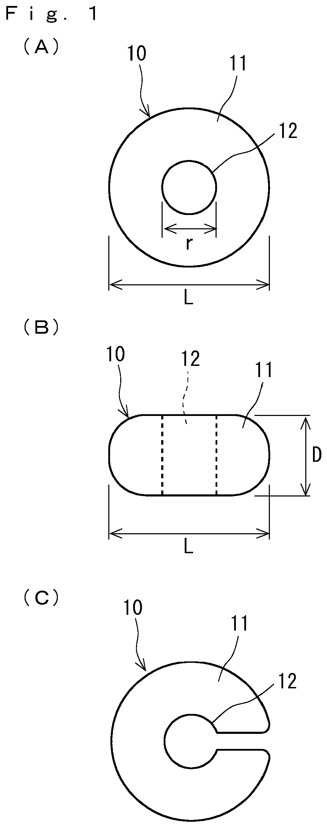

Here, the "ball shape" is not limited to a true sphere, but means a sphere, an ellipsoid, or a shape similar thereto. As shown in Fig. 1, the ball member 11 is formed, for example, as one lump rounded without corner parts except for an edge of an opening of a through-hole 12. An outer periphery of the ball member 11 in a front view is constituted only by curves (see Fig. 1(A)), and the outer periphery of the ball member 11 is rounded in a cross-sectional view over the entire length.

Specifically, the ball member 11 is formed in an oblate spheroid shape. The ball member 11 exhibits a flat oblate spheroid shape whose short axis extends in a thickness direction. The ball member 11 is formed such that a minimum dimension L of an outer shape in the front view shown in Fig. 1(A) is larger than a thickness dimension D (see Fig. 1(B)). In the present embodiment, since the shape of the ball member 11 in the front view is circular, the minimum dimension L of the outer shape is a diameter of the ball member 11. When the shape of the ball member 11 in the front view is vertically long, the minimum dimension L of the outer shape is a lateral dimension (width dimension) orthogonal to a longitudinal direction. For example, the minimum dimension L of the outer shape can be set to 1.5 times or more of the thickness dimension D. In a third modification described later, the minimum dimension L of the outer shape is set to 3 times or more of the thickness dimension D. By setting such a shape, in a front surface and a back surface of the ball member 11, when there is a substantially flat region, an area of the region becomes large, and when there is no substantially flat region, an area of a region having a small curvature becomes large, and a contact area with the bone 8 becomes large.

In the ball member 11, the through-hole 12 extending in the thickness direction (short axis direction) is formed as a penetrating part through which the medical wire 5 penetrates. The ball member 11 exhibits a ring shape (or a donut shape) by having the through-hole 12. The through-hole 12 can be, for example, a circular hole. The ball member 11 functions as a hollow rivet. Note that the penetrating part 12 may have an opening part on a side surface side of the ball member 11 as shown in Fig. 1(C). In the front view, the ball member 11 exhibits a substantially C-shape.

Regarding dimensions of the ball member 11, a plurality of dimensions is standardized so that they can be selectively used according to a diameter of the medical wire 5. As the medical wire 5, one having 0.7 mm to 2.4 mm is generally used. The minimum dimension (diameter in the present embodiment) L of the outer shape of the ball member 11 in the front view can be set to, for example, 1.5 mm to 8 mm. The thickness D of the ball member 11 can be set to, for example, 1 mm to 5 mm. A diameter r of the through-hole 12 is made slightly larger than the diameter of the medical wire 5, and can be set to, for example, 0.8 mm to 2.8 mm.

### [Usage Method of Wire Fixture]

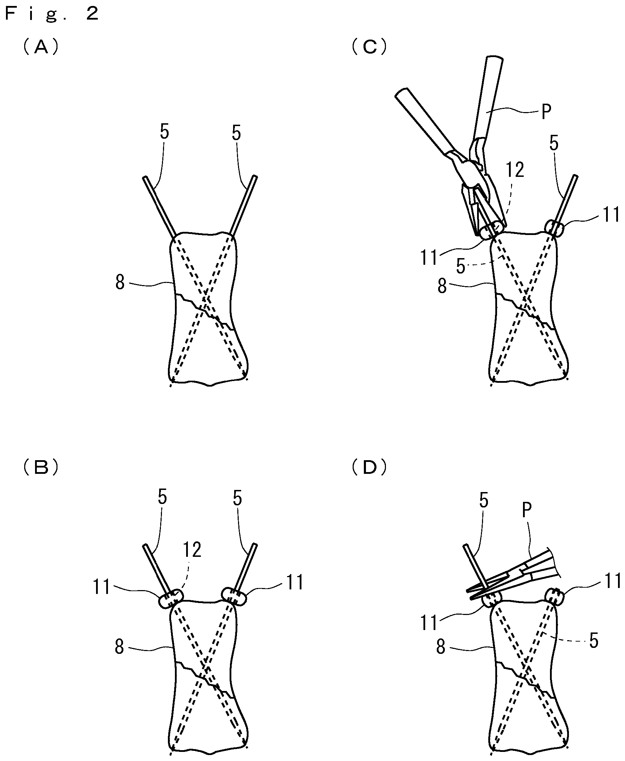

Subsequently, referring to Fig. 2, description will be given regarding a usage method of the wire fixture 10. The wire fixture 10 is used from a state where an insertion work of the medical wire 5 into the bone 8 is completed (a state shown in Fig. 2(A)). Hereinafter, description will be given by taking a case of the open reduction and internal fixation method as an example.

First, the medical wire 5 is passed through the through-hole 12 of the ball member 11, and as shown in Fig. 2(B), the ball member 11 is moved to a position where the back surface (or the front surface) of the ball member 11 contacts the outer surface of the bone 8. Then, in this state, as shown in Fig. 2(C), the ball member 11 is attached (fixed) to the medical wire 5 by gripping the side surface of the ball member 11 using a tool P such as pliers, crushing the ball member 11 from this state, and sandwiching the medical wire 5 by the inner surface (inner surfaces facing each other) of the through-hole 12 (that is, by caulking the ball member 11). The ball member 11 serves as a "barb".

Furthermore, after attachment of the ball member 11, as shown in Fig. 2(D), using the tool P such as pliers, a base of a portion of the medical wire 5 protruding from the through-hole 12 of the ball member 11 is cut. That is, the medical wire 5 is cut immediately above the ball member 11. Then, after a series of these works, by performing suture of the skin or the like, the surgery is completed. Note that illustration of the skin is omitted in Fig. 2.

Note that in the case of the percutaneous pinning method, at a position where the back surface (or the front surface) of the ball member 11 contacts the outer surface of the skin, using the tool P such as pliers, the ball member 11 is attached to the medical wire 5 by crushing the ball member 11 and sandwiching the medical wire 5 by the inner surface of the through-hole 12,. Then, when the medical wire 5 is cut immediately above the ball member 11, the treatment is completed.

### [Regarding Effects and the Like of Present Embodiment]

In the present embodiment, the ball member 11 is attached to the medical wire 5 by a simple work of caulking the ball member 11, and it is possible to easily fix the ball member 11 at a position just at the limit of the insertion part (a position where the ball member 11 contacts the outer surface of the bone 8 or the outer surface of the skin). The position of the medical wire 5 is fixed by the ball member 11. A risk that the medical wire 5 shifts from an initial position where it was inserted is reduced. Moreover, when the ball member 11 is exposed to the body surface, since the medical wire 5 is cut so that a cut end of the medical wire 5 hardly protrudes from the ball member 11, a risk that the ball member 11 gets caught and the medical wire 5 comes out is also reduced. According to the present embodiment, it is possible to prevent a decrease in fixing force for fixing the bone 8 and a failure of the fixed bone 8 caused by positional displacement of the medical wire 5.

Moreover, in the present embodiment, even if a soft tissue such as a blood vessel, a nerve, a tendon, or the like exists in the vicinity of the attachment location of the ball member 11, the ball member 11 is formed in a ball shape, and damage is less likely to occur1 in the soft tissue even if the ball member 11 contacts the soft tissue.

Here, when fixation of the position of the medical wire 5 is not performed by the ball member 11, since there are various concerns described in [Background Art], removal of the medical wire 5 may be performed as early as possible in a state where fusion of the bone 8 is obtained to some extent. On the other hand, in the present embodiment, since the risk of movement of the medical wire 5 is low and there are no other concerns, fixation of the bone 8 by the medical wire 5 can be performed over a period during which fixation of the bone 8 is necessary.

### [Modification 1 of Embodiment]

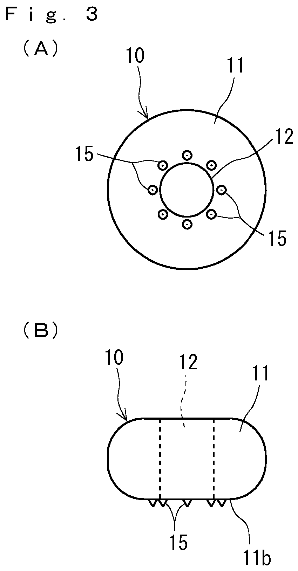

In this modification, as shown in Fig. 3, a plurality of protrusions 15 having sharp tips are formed on a back surface 11b (a surface where one end of the through-hole 12 opens) of the ball member 11. The plurality of protrusions 15 are arranged in the vicinity of the through-hole 12 and arranged along a circumferential direction of the through-hole 12. Each protrusion 15 is formed in a tack-like shape. Dimensions of each protrusion 15 can be set to about 1 mm in diameter (diameter of a bottom part) and about 1 mm in height. The number of the protrusions 15 can be set to, for example, 3 or more.

In this modification, the medical wire 5 is passed through the through-hole 12 of the ball member 11 so that the back surface 11b of the ball member 11 faces the bone 8 side. Further, after the ball member 11 is caulked and attached to the medical wire 5, a work of pushing the ball member 11 toward the bone 8 side and driving each protrusion 15 into the bone 8 is performed.

Here, when inserting the medical wire 5, there is a case where the medical wire 5 deflects. In such a case, if the medical wire 5 rotates, it affects stability of the fixed bone 8. In this modification, rotation of the medical wire 5 is suppressed by each protrusion 15. Therefore, it is possible to suppress an influence on stability of the fixed bone 8.

### [Modification 2 of Embodiment]

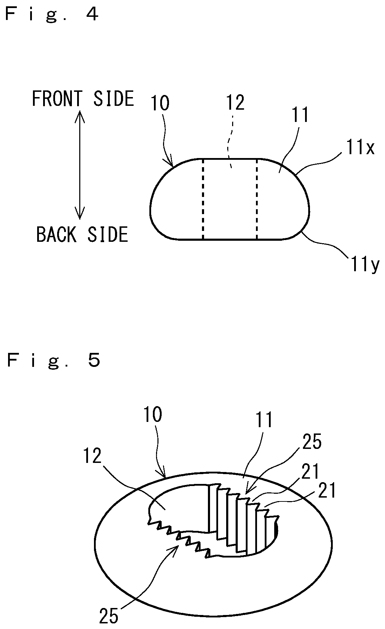

In this modification, as shown in Fig. 4, a shape of the ball member 11 in a side view is different from that of the embodiment. In the ball member 11, a curvature of an outer periphery 11y on the back side is larger than a curvature of an outer periphery 11x on the front side. Therefore, the ball member 11 has a large contact area with the bone 8.

### [Modification 3 of Embodiment]

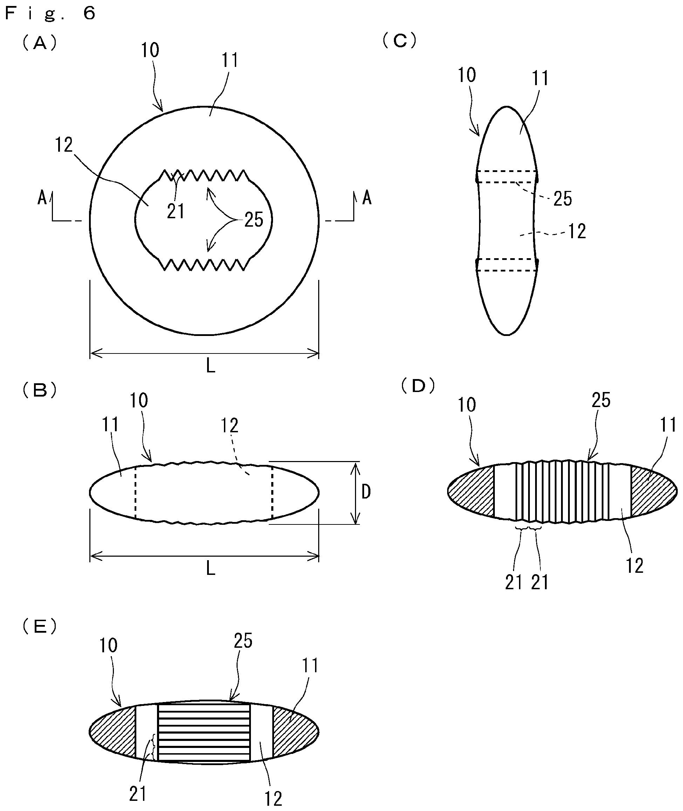

In this modification, one or a plurality of convex parts 21 protruding from the inner surface of the through-hole 12 of the ball member 11 are provided. In this modification, as shown in Fig. 5 and Figs. 6(A)-(D), a concave-convex part 25 composed of a plurality of continuous convex parts 21 (for example, three or more convex parts 21) is formed on the inner surface of the through-hole 12. Note that in this modification, the penetrating part 12 may have an opening part on the side surface side of the ball member 11 as shown in Fig. 1(C).

In the front view of the through-hole 12 (ball member 11) shown in Fig. 6(A) (viewed in a penetrating direction of the through-hole 12), each convex part 21 in the concave-convex part 25 is formed in a tapered shape (for example, a triangular shape whose base is on a root side), and a tip of each convex part 21 is sharp. The concave-convex part 25 exhibits a jagged shape. Further, on the inner surface of the through-hole 12, each convex part 21 is formed throughout the penetrating direction of the through-hole 12 (thickness direction of the ball member 11).

On the inner surface of the through-hole 12, a pair of concave-convex parts 25 are formed in regions facing each other in the front view of the through-hole 12. Here, the through-hole 12 is formed to be horizontally elongated in the front view shown in Fig. 6(A). A front shape of the through-hole 12 exhibits a shape having a longitudinal direction and a widthwise direction intersecting the longitudinal direction (for example, a direction orthogonal to the longitudinal direction). A dimension in the widthwise direction of the through-hole 12 is made slightly larger than the diameter of the medical wire 5. The pair of concave-convex parts 25 face each other in the widthwise direction of the penetrating part 12 in the front view.

When using the wire fixture 10, the ball member 11 is crushed in the widthwise direction of the through-hole 12 in a state where the medical wire 5 penetrates the through-hole 12. The medical wire 5 is sandwiched by the pair of concave-convex parts 25. Thereby, positional displacement of the ball member 11 with respect to the medical wire 5 is less likely to occur. Note that in the concave-convex part 25 of this modification, the plurality of convex parts 21 are arranged continuously in the circumferential direction of the through-hole 12, but as shown in Fig. 6(E), the plurality of convex parts 21 may be arranged continuously in the penetrating direction of the through-hole 12. In this case, positional displacement of the ball member 11 in a length direction of the medical wire 5 is less likely to occur.

### [Modification 4 of Embodiment]

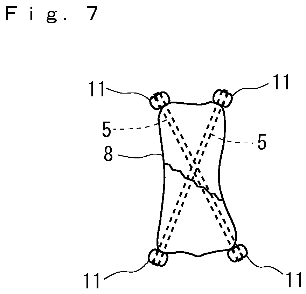

In this modification, as shown in Fig. 7, the ball members 11 are attached to both the insertion part side and a opposite side of the medical wire 5. The wire fixture 10 includes two ball members 11 for one medical wire 5. In this modification, the position of the medical wire 5 is more securely fixed.

### INDUSTRIAL APPLICABILITY

The present invention is applicable to a wire fixture used in the medical field and the like.

### DESCRIPTION OF REFERENCE CHARACTERS

- 5: Medical wire
- 8: Bone
- 10: Wire fixture
- 11: Ball member
- 12: Through-hole (Penetrating part)

## Claims

1. A wire fixture for fixing a position of a medical wire inserted into a bone, the wire fixture being configured to be attached to the medical wire, the wire fixture comprising:
a ball member formed in a ball shape and having, as a portion through which the medical wire penetrates, a penetration portion extending in a thickness direction of the ball member, and
wherein, the ball member is configured to be crushed in a state that the medical wire penetrates the penetration portion so that the medical wire is clamped by an inner surface of the penetration portion and the ball member is attached to the medical wire.

2. The wire fixture according to claim 1, wherein one or more convex portions are formed on the inner surface of the penetration portion.

3. The wire fixture according to claim 1, wherein a pair of concave-convex parts are formed on the inner surface of the penetration portion in regions facing each other in a front view of the penetration portion (as viewed in an extending direction of the penetration portion).

4. The wire fixture according to claim 3, wherein the pair of concave-convex parts face each other in a widthwise direction of the penetration portion in the front view.

5. The wire fixture according to claim 1, wherein a plurality of protrusions each having a pointed tip are formed on a back surface of the ball member where one end of the penetration portion opens.

6. The wire fixture according to claim 1, wherein a thickness dimension of the ball member is smaller than a minimum outer dimension of the ball member in a front view.

7. The wire fixture according to claim 6, wherein the ball member is formed in an oblate spheroid shape, and the penetration portion is a penetration hole extending in a widthwise direction of the ball member.
